(19) Europäisches Patentamt
European Patent Office
Office européen des brevets

(11) EP 1 558 755 B1

(12) FASCICULE DE BREVET EUROPEEN

(45) Date de publication et mention de la délivrance du brevet:
**24.10.2012 Bulletin 2012/43**

(21) Numéro de dépôt: **03772376.4**

(22) Date de dépôt: **22.09.2003**

(51) Int Cl.:
***C12Q 1/527*** *(2006.01)*

(86) Numéro de dépôt international:
**PCT/FR2003/002782**

(87) Numéro de publication internationale:
**WO 2004/027087 (01.04.2004 Gazette 2004/14)**

# (54) METHODE DE DETERMINATION DE GROUPEMENTS SPECIFIQUES CONSTITUANT LES HEPARINES

VERFAHREN ZUR BESTIMUNG VON SPEZIFISCHEN GRUPPEN VON HEPARINE

METHOD OF DETERMINING SPECIFIC GROUPS FORMING HEPARINS

(84) Etats contractants désignés:
**AT BE BG CH CY CZ DE DK EE ES FI FR GB GR HU IE IT LI LU MC NL PT RO SE SI SK TR**

(30) Priorité: **23.09.2002 FR 0211724**
**31.10.2002 US 422482 P**

(43) Date de publication de la demande:
**03.08.2005 Bulletin 2005/31**

(73) Titulaire: **Aventis Pharma S.A.**
**92160 Antony (FR)**

(72) Inventeurs:
- **MOURIER, Pierre**
  **F-94220 Charenton Le Pont (FR)**
- **VISKOV, Christian**
  **F-91130 Ris Orangis (FR)**

(74) Mandataire: **Jacobson, Claude**
**Cabinet Lavoix**
**2, Place d'Estienne d'Orves**
**75441 Paris Cedex 09 (FR)**

(56) Documents cités:
**WO-A-88/02400 US-B1- 6 190 875**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).



Printed by Jouve, 75001 PARIS (FR)

## Description

La présente invention a pour objet une méthode d'analyse de groupements spécifiques constituant les héparines ou les héparines de bas poids moléculaire.

Lors du procédé de préparation de l'Enoxaparine (Lovenox®) (US5,389,618) à partir de l'héparine pure, L'étape de procédé de dépolymérisation alcaline en phase aqueuse produit une transformation partielle mais caractéristique des glucosamines des terminaisons réductrices des chaînes oligosaccharidiques.

La première étape de cette transformation consiste en une épimerisation glucosamine↔mannosamine (T. Toida and al, J. Carbohydrate Chemistry, 15(3), 351-360 (1996)); la deuxième étape est une 6-O desulfatation de la glucosamine conduisant à la formation de dérivés nommés « 1,6 anhydro » (demande de brevet internationale WO01/29055).

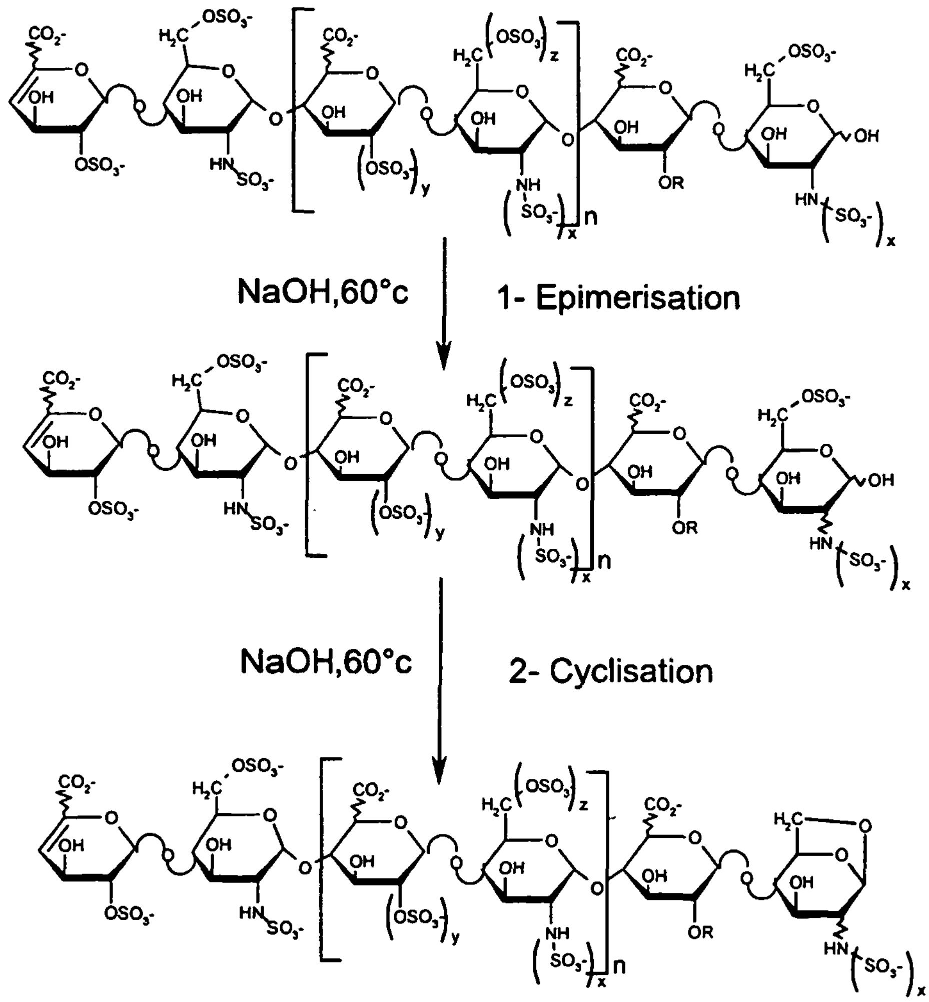


Ce type de dérivé n'est obtenu que pour les chaînes oligosaccharidiques dont la glucosamine terminale est 6-O sulfatée.

Le pourcentage de chaînes oligosaccharidiques dont la terminaison est modifiée par une liaison 1,6-anhydro est une caractéristique structurale du mélange d'oligosaccharide du Lovenox et doit pouvoir être mesuré.

La présente invention consiste donc en une méthode d'analyse des héparines, des héparines de bas poids moléculaire et plus particulièrement du Lovenox.

La méthode d'analyse selon l'invention est la suivante :

L'échantillon à doser est dépolymérisé par action d'héparinases puis le cas échéant le dépolymérisat obtenu est réduit puis on effectue une analyse par chromatographie liquide Haute Performance.

La méthode telle que définie plus haut est donc caractérisée en ce que l'on recherche la présence de chaînes oligosaccharides dont la terminaison est modifiée par une liaison 1,6-anhydro (« Groupements 1,6 anhydro »).

En particulier, l'échantillon à doser est tout d'abord dépolymérisé de façon exhaustive par un mélange d'héparinases et notamment d'héparinase 1 (EC 4.2.2.7.), d'héparinase 2 (heparin Lyase II) et d'héparinase 3 (EC4.2.2.8.)).

(Ces enzymes sont commercialisées par la société Grampian Enzymes). La demande internationale W088/02400 décrit une méthode de dépolymérisation de l'héparine par une héparinase et neutralisation de l'activité anticoagulante de l'héparine contenue dans une échantillon sanguin.

L'invention a donc pour objet une méthode d'analyse des héparines ou des héparines de bas poids moléculaire pour rechercher la présence de chaînes oligosaccharides dont la terminaison est modifiée par une liaison 1,6-anhydro, caractérisée en ce qu'on effectue les étapes suivantes :

1) dépolymérisation de l'échantillon par action d'héparinases,
2) réduction du dépolymérisat,
3) dosage par Chromatographie liquide Haute Performance, la méthode chromatographique utilisée étant une chromatographie par échange d'anion pour la détermination des groupements 1,6-anhydro, dans laquelle :

- on utilise une phase mobile transparente dans l'UV jusqu'à 200 nm et
- une double détection à 234 nm d'une part et 202-230nm d'autre part permettant de détecter sélectivement les sucres acétylés, la dite méthode s'effectuant en prenant comme signal, pour la détection sélective des sucres acétylés, la différence entre l'absorbance à ces 2 longueurs d'onde (202 et 230 nm) choisies de telle manière que l'absorptivité des saccharides non acétylés s'annule,

et caractérisée en ce que les résidus 1,6-anhydro obtenus lors de la réaction de dépolymérisation sont les suivants :

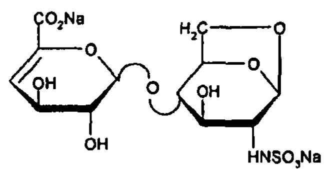

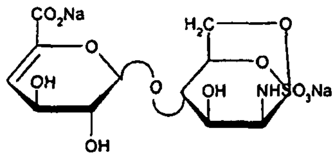

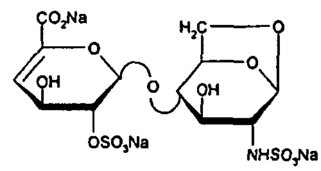

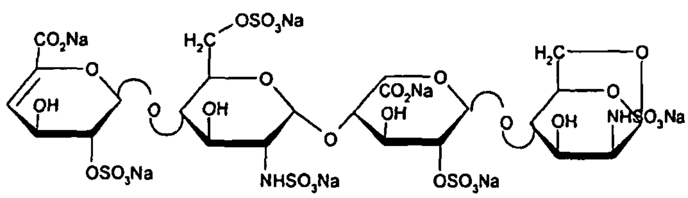

les héparinases sont sous forme d'un mélange d'héparinase 1 (EC 4.2.2.7.), d'héparinase 2 (heparin lyase II) et d'héparinase 3 (EC.4.2.2.8.)

Le dépolymérisat ainsi préparé est ensuite traité de préférence par une solution de $NaBH_4$ dans l'acétate de sodium. Cette dernière opération permet de réduire spécifiquement les extrémités réductrices qui ne sont pas sous la forme 1,6 anhydro (produits décrit dans la demande de brevet WO 01/72762). Enfin, pour pouvoir quantifier les disaccharides 1 et 2 décrits plus bas, l'échantillon d'héparine de bas poids moléculaire, dépolymérisé par les héparinases, doit être réduit par l'action d'un agent réducteur tel que $NaBH_4$.

L'invention a donc plus particulièrement pour objet la méthode telle que définie plus haut caractérisée en ce que l'héparine dépolymérisée est ensuite réduite.

L'invention a tout particulièrement pour objet la méthode telle que définie précédemment caractérisée en ce que l'agent de réduction est le $NaBH_4$. On pourra éventuellement utiliser un autre sel de métal alcalin du borohydrure tel que le lithium ou le potassium.

Le dosage des terminaisons 1,6 anhydro est ensuite réalisé par CLHP (Chromatographie Liquide Haute Performance) et en particulier par chromatographie échangeuse d'anions.

La méthode de dosage selon l'invention permet de bien différencier le Lovenox des autres héparines de bas poids moléculaire qui ne renferment pas ces dérivés « 1,6-anhydro ». Inversement, la méthode de dosage selon l'invention permet de s'assurer que des héparines de bas poids moléculaire ne remplissent pas les caractéristiques physicochimiques du Lovenox et donc sont de nature différente.

La méthode de dosage selon l'invention peut-être appliquée au procédé industriel lors des contrôles d'échantillons au cours du procédé, afin d'assurer une standardisation du procédé de fabrication du Lovenox et obtenir des lots uniformes.

Après dépolymérisation enzymatique et réduction des extrémités réductrices, on trouve les dérivés 1,6-anhydro du Lovenox sous 4 formes essentielles.

Tous les oligosaccharides ou polysaccharides qui comportent l'extrémité 1,6-anhydro sur l'unité disaccharidique terminale et qui ne possèdent pas un 2-0 sulfate sur l'acide uronique, du dit disaccharide terminal, sont totalement dépolymérisés par les héparinases et sous la forme des disaccharides 1 et 2. Par contre, Lorsque le dit saccharide terminal comporte un 2-0 sulfate sur l'acide uronique et qu'il est sous la forme mannosamine, le dérivé 1,6 anhydro se retrouve sous la forme du tétrasaccharide 1 (forme résistante aux héparinases).

Le trisaccharide 1 (voir plus bas), est également présent dans le mélange. Il est issu d'un autre processus de dégradation qui conduit à la structure ci-après (phénomène de peeling observé dans lors de la dépolymérisation chimique du Lovenox.

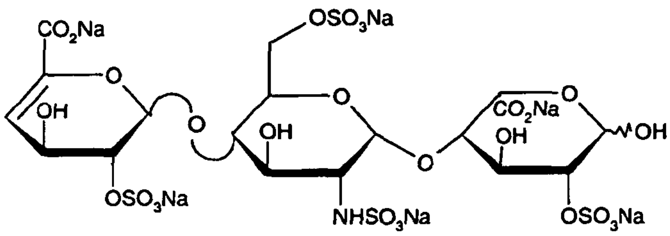

trisaccharide 1

Les autres constituants du mélange ne sont pas caractéristiques uniquement du Lovenox. On trouve bien entendu les 8 disaccharides élémentaires de la chaîne héparinique. Ces 8 disaccharides élémentaires sont commercialisés entre autre par la société Sigma.

D'autres disaccharides ont été identifiés dans le mélange par la méthode selon l'invention: les disaccharides $\Delta IIs_{gal}$ et $\Delta IVs_{gal}$ qui ont pour origine la 2-0 desulfatation alcaline de -IdoA(2S)-GlcNS(6S)- et de -IdoA(2S)-GlcNS- conduisant à la formation de 2 acides galacturoniques. Il ne sont pas habituellement présents dans la structure originelle de l'héparine (U.M. Desai et Coll. Arch. Biochem. Biophys., 306 (2) 461-468 (1993).

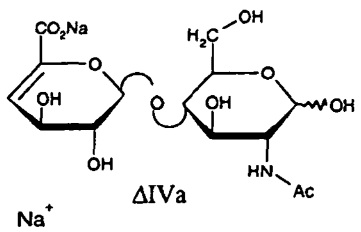

ΔIVa

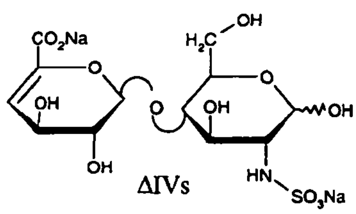

ΔIVs

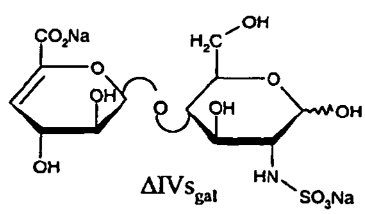

$\Delta IVs_{gal}$

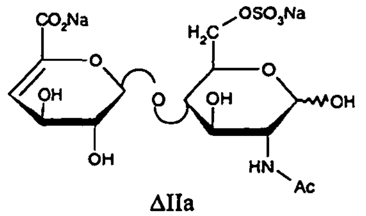

ΔIIa

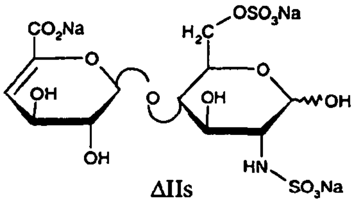

ΔIIs

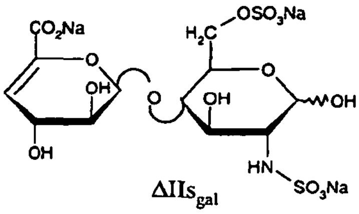

$\Delta IIs_{gal}$

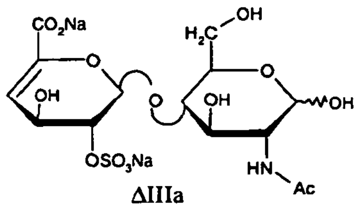

ΔIIIa

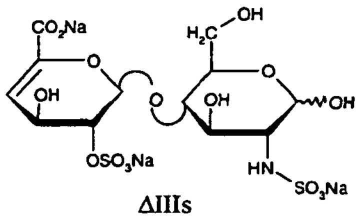

ΔIIIs

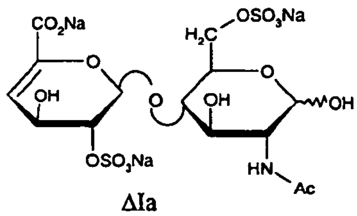

ΔIa

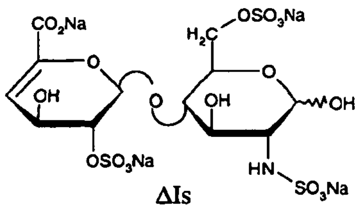

ΔIs

Les oligosaccharides comportant des glucosamines 3-0 sulfatés résistent au clivage par les héparinases et restent présents sous forme de tétrasaccharides.

Dans le cas de la plupart des héparines de bas poids moléculaire, l'héparine est extraite du mucus de porc, et ces tétrasaccharides principaux sont représentés plus bas. Ils sont resistants à la dépolymérisation enzymatique et sont le reflet des séquences affines à l'antithrombine III. Ils sont symbolisés ainsi : $\Delta$IIa-$\underline{IIs}_{glu}$ et $\Delta$IIa-$\underline{IVs}_{glu}$. (S.YAMADA, K.YOSHIDA, M. SUGIURA, K.SUGAHARA, K-H KHOO, H.R. MORRIS, A. DELL, J.Biol.Chem. ; 270(7), 4780-4787 (1993)

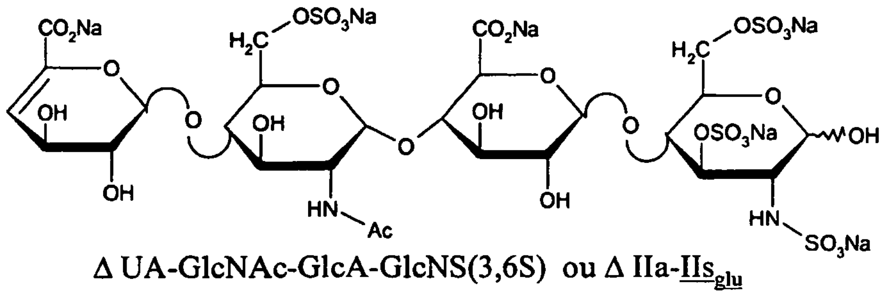

Δ UA-GlcNAc-GlcA-GlcNS(3,6S) ou Δ IIa-$\underline{IIs}_{glu}$

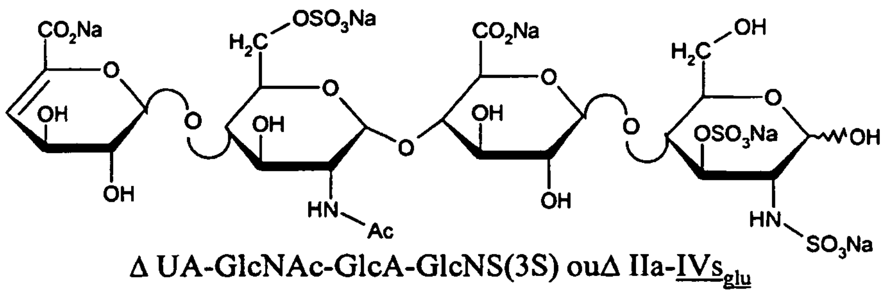

Δ UA-GlcNAc-GlcA-GlcNS(3S) ouΔ IIa-$\underline{IVs}_{glu}$

Le dernier constituant du mélange clivé par les héparinases est la terminaison glycosérine ΔGlcA-Gal-Gal-Xyl-Ser (K.SUGAHARA, H.TSUDA, K.YOSHIDA, S.YAMADA, J.Biol.Chem. ; 270(39), 22914-22923 (1995) ; K.SUGAHARA, S.YAMADA, K.YOSHIDA,P. de WAARD, J.F.G. VLIEGENTHART ; J.Biol.Chem. ; 267(3), 1528-1533 (1992). Ce dernier est généralement presque absent du Lovenox (Voir RMN à l'exemple 5).

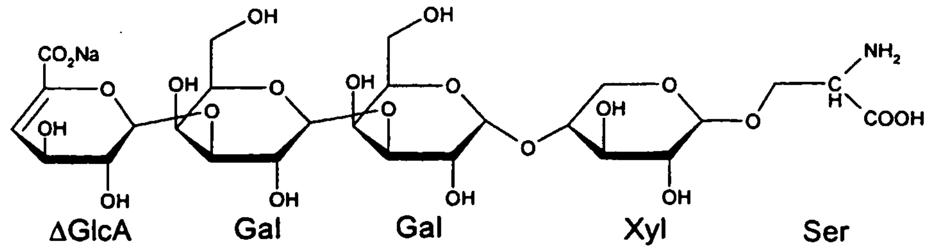

Un autre aspect de l'invention se situe au niveau du procédé de chromatographie utilisé pour la détermination des groupements 1,6-anhydro. Tout d'abord il s'agit de séparer les différents polysaccharides obtenus après dépolymérisation et traitement par un agent réducteur tel que le $NaBH_4$.

La chromatographie d'échange d'anions (SAX) est la méthode séparative la plus adaptée à un tel mélange complexe.

Les colonnes remplies d'une phase stationnaire du type Spherisorb SAX de granulométrie 5 $\mu$m et d'une longueur de 25 cm peuvent être utilisées. Tous les diamètres de colonne classiques, compris entre 1mm et 4.6 mm sont utilisables.

L'appareillage utilisé peut-être un chromatographe permettant la formation de gradient d'élution avec un détecteur UV, plus préférablement muni d'une barrette de diodes afin de pouvoir réaliser des spectres UV des constituants et d'enregistrer des signaux complexes, résultant de la différence entre les absorbances à 2 longueurs d'ondes différentes et permettant la détection spécifiques des oligosaccharides acétylés. Pour permettre ce type de détection, des phases mobiles transparentes dans l'UV jusqu'à 200 nm sont préférables. Ceci exclut les phases mobiles classiques à base de NaCl qui ont par ailleurs l'inconvénient de nécessiter un chromatogramme passivé afin de résister au pouvoir corrosif des chlorures. La phase mobile utilisée ici sera de préférence à base de perchlorate de sodium, mais les sels de méthane sulfonate ou phosphate peuvent aussi être utilisés.

Le pH préconisé pour la séparation est de 2 à 6,5. Préférentiellement, on utilisera un pH voisin de 3. Il est contrôlé ici par addition d'un sel tel que le phosphate possédant un pouvoir tampon à pH = 3 meilleur que celui des perchlorates.

A titre d'exemple, on donne ci-après des conditions standard de séparation chromatographique :

Solvant A : $NaH_2PO_4$ 2,5mM porté à pH 2,9 par addition de $H_3PO_4$
Solvant B : $NaClO_4$ 1N- $NaH_2PO_4$ 2,5mM porté à pH 3,0 par addition de $H_3PO_4$

Le gradient d'élution peut être le suivant :

T=0min : %B = 3 ; T= 40 min: %B = 60 ; T = 60 min : %B = 80

La présente invention a donc également pour objet une méthode d'analyse telle que définie plus haut par séparation par chromatographie par échange d'anions caractérisée en ce qu'on utilise la phase mobile transparente dans l'UV jusqu'à 200nM.

L'invention a plus particulièrement pour objet une phase mobile telle que définie plus haut à base de perchlorate de sodium, de sels de méthane sulfonate ou de sels de phosphate.

Un autre aspect tout à fait important se trouve dans la méthode de détection.

Une méthode a été développée afin d'accroître la spécificité de la détection UV. Comme les polysaccharides non acétylés ont tous, à un pH donné, un spectre UV assez proche, il est possible de détecter sélectivement les sucres acétylés en prenant comme signal la différence entre l'absorbance à 2 longueurs d'onde choisies de telle manière que l'absorptivité des saccharides non acétylés s'annule.

Dans le cas ci-dessous, on choisira 202 nm et 230 nm comme longueurs d'onde de détection et de référence et l'on notera le signal 202 - 230 nm. Le choix est bien entendu dépendant du pH de la phase mobile (des ajustements de quelques nm peuvent être nécessaires pour être à l'optimum des dites conditions). Le détecteur le plus adapté à cette technique est le détecteur DAD 1100 de la société Agilent Technologies. Dans ce cas, une double détection sera réalisée à 234 nm d'une part, et à 202-230 nm d'autre part. Le principe de détection sélective des oligosaccharides acétylés est illustré sur la figure ci-dessous dans laquelle le spectre UV d'un disaccharide sulfaté Delta 1s est comparé avec celui d'un disaccharide acétylé Delta 1a

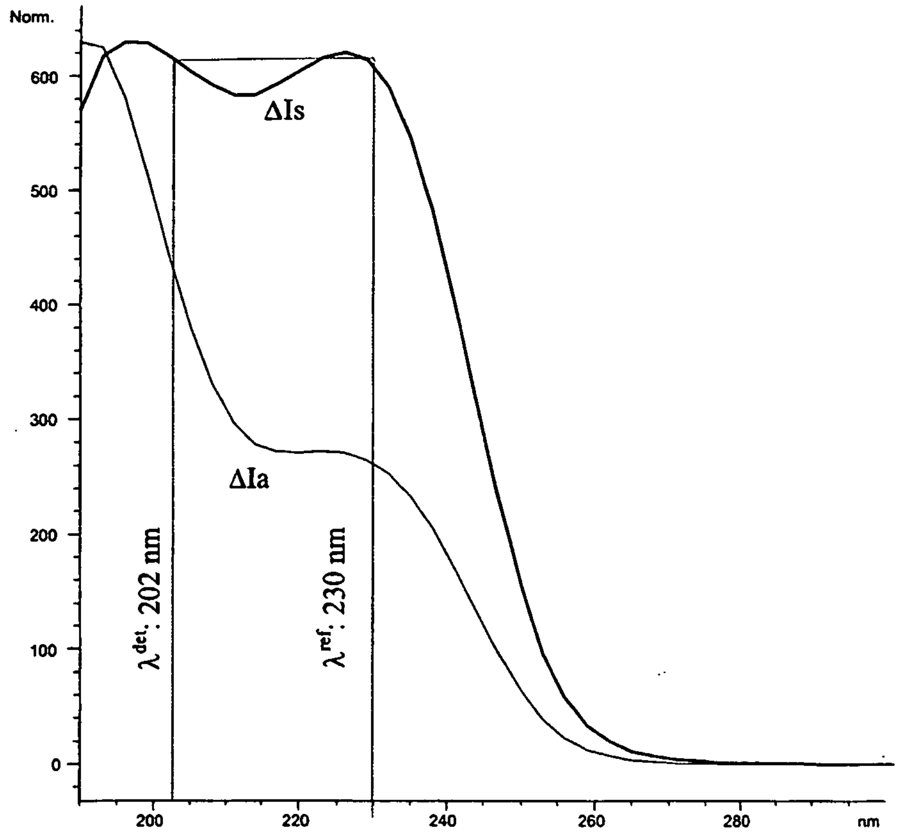


La présente invention a donc également pour objet une méthode d'analyse telle que définie plus haut par séparation par chromatographie par échange d'anions caractérisée en ce que la méthode de détection permet de détecter sélectivement les sucres acétylés.

L'invention a également tout particulièrement pour objet une méthode d'analyse telle que définie plus haut par séparation par chromatographie par échange caractérisé en ce que la détection sélective des sucres acétylés s'effectue en prenant comme signal la différence entre l'absorbance à 2 longueurs d'onde choisies de telle manière que l'absorptivité des saccharides non acétylés s'annule.

La quantification des 4 résidus 1,6-anhydro décrits plus haut nécessite une sélectivité suffisante du système chromatographique vis à vis de tous les autres constituants du mélange. Or, les 2 disaccharides 1 et 2, en général co-élués, sont mal résolus par rapport à ΔIIa, surtout que ce dernier est présent sous la forme de ses 2 anomères α et β.

L'identité des 2 disaccharides 1 et 2 peut être facilement vérifiée car ils se forment en quelques heures à température ambiante dans une solution aqueuse de ΔIIs portée à pH 13 par addition de NaOH. Cependant, si la double détection est utilisée, les oligosaccharides acétylés ΔIVa, ΔIIa, ΔIIIa, ΔIa, ΔIIa-$\underline{IVs}_{glu}$ et ΔIIa-$\underline{IIs}_{glu}$ sont facilement identifiables.

Les causes de dédoublement des pics sont les formes anomères d'une part, et dans une moindre mesure l'épimérisation glucosamine ↔ mannosamine présente partiellement pour ΔIIs, ΔIIIs et ΔIs lorsqu'ils sont en position terminale dans la chaîne oligosaccharidique.

Pour pouvoir quantifier les disaccharides 1 et 2, l'échantillon d'héparine de bas poids moléculaire, dépolymérisé par les héparinases, est réduit par l'action $NaBH_4$.

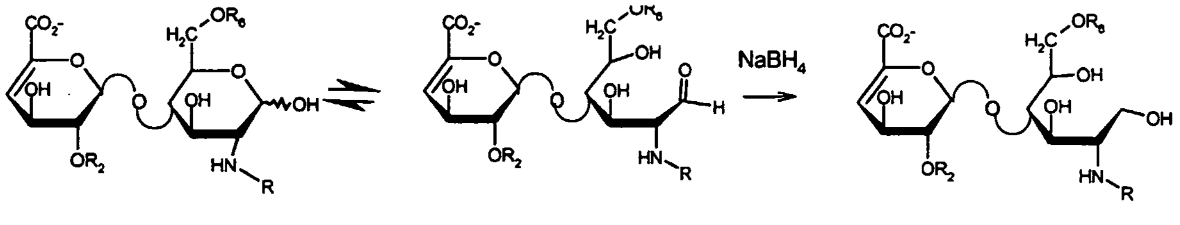


Anomère α + Anomère β

Cette réduction a pour avantage de supprimer les anoméries $\alpha \leftrightarrow \beta$ par ouverture du cycle oligosaccharidique terminal. Le chromatogramme obtenu est plus simple puisque les anoméries sont supprimées et surtout la réduction de ΔIIa diminue sa rétention sur la colonne et permet un dosage facile des disaccharides 1 et2.

Les exemples de chromatogrammes décrits dans les figures 1 et 2 ci-après illustrent bien ces phénomènes et les avantages de cette méthode.

Enfin, l'invention a également pour objet les dérivés saccharidiques nouveaux obtenus par la mise en oeuvre du procédé de dépolymérisation et de réduction choisis parmi le disaccharide 1, le disaccharide 2, le disaccharide 3 et le Trisaccharide 1.

Les exemples ci-dessous illustrent l'invention sans toutefois avoir un caractère limitatif

Exemple 1 :

La dépolymérisation enzymatique est réalisée pendant 48heures à température ambiante en mélangeant 50 µl d'une solution à 20 mg/ml de l'héparine de bas poids moléculaire à doser, 200 µl dune solution 100mM acide acétique/NaOH à pH 7.0 contenant 2 mM d'acétate de calcium et 1 mg/ml de BSA avec 50 µl de la solution mère des 3 héparinases.

La réduction est réalisée sur 60µl du produit dépolymérisé par les héparinases en rajoutant 10 µl d'une solution de $NaBH_4$ à 30 g/l dans l'acétate de sodium 100 mM préparée extemporanément. On notera que les héparinases sont conservées à - 30°C. Les héparinases sont en solution tampon et leur titre est de 0.5 UI/ml (Composition de la solution tampon : solution aqueuse pH 7 de $KH_2PO_4$ à une concentration 0.01 mole/l et additionnée de sérum d'albumine bovine (BSA) à 2 mg/ml).

**Exemple 2 :**

RMN du Disaccharide 3 obtenu selon le procédé décrit plus haut

Spectre proton dans $D_2O$, 400 MHz, T=298K, δ en ppm: 3,34 (1H, dd, J=7 et 2Hz, H2), 3,72 (1H, t, J=8Hz, H6), 3,90 (1H, m, H3), 4,03 (1H, s, H4), 4,20 (1H, d, J=8Hz, H6), 4,23 (1H, t, J=5Hz, H3'), 4,58 (1H, m, H2'), 4,78 (1H, m, H5), 5,50 (1H, s, H1), 5,60 (1H, dd, J=6 et 1Hz, H1'), 6,03 (1H, d, J=5Hz, H4')].

**Exemple 3**

RMN du Tetrasaccharide 1 obtenu selon le procédé décrit plus haut.

Spectre proton dans $D_2O$, 400 MHz, T=298K, δ en ppm: 3,15 (1H, s, H2), 3,25 (1H, m, H2''), 3,60 (1H, m, H3''), entre 3,70 et 4,70 (14H, massif, H3/H4/H6, H2'/H3'/H4'/H5', H4''/H5''/H6'', H2'''/H3'''), 4,75 (1H, m, H5), entre 5,20 et 5,40 (2H, m, H1' et H1''), 5,45 (1H, m, H1'''), 5,56 (1H, m, H1), 5,94 (1H, d, J=5Hz, H4)

**Exemple 4 :**

RMN du Trisaccharide 1 obtenu selon le procédé décrit plus haut.

Spectre dans $D_2O$, 600 MHz (δ en ppm) : 3,28 (1H, m), 3,61 (1H, t, 7Hz), 3,79 (1H, t, 7Hz), 3,95 (1H, d, 6Hz), 4,00 (1H, s), 4,20 (1H, m), 4,28 (2H, m), 4,32 (1H, d, 4Hz), 4,41 (1H, s), 4,58 (1H, s), 4,61 (1H, s), 4,90 (1H, s large), 5.24 (1H, s), 5,45 (1H, s), 5,95 (1H, s).

**Exemple 5 :**

RMN du ΔGlcA-Gal-Gal-Xyl-Ser

Spectre dans $D_2O$, 500 MHz (δ en ppm): 3,30 (1H, t, 7Hz), 3,34 (1H, t, 8Hz), 3,55 (1H, t, 7Hz), 3,60 (1H, t, 7Hz), entre 3,63 et 3,85 (10H, m), 3,91 (2H, m), 3,96 (1H, dd, 7 et 2Hz), entre 4,02 et 4,10 (3H, m), 4,12 (1H, d, 2Hz), 4,18 (1H, m), 4,40 (1H, d, 6Hz), 4,46 (1H, d, 6Hz), 4,61 (1H, d, 6Hz) , 5,29 (1H, d, 3Hz), 5,85 (1H, d, 3Hz).

**Exemple 6 : Principe de la quantification**

Dans la méthode selon l'invention, il est fait comme hypothèse, largement admise, que tous les oligosaccharides insaturés contenus dans le mélange ont la même absorptivité molaire, égale à 5500 $mole^{-1}.l.cm^{-1}$.

Il est donc possible de déterminer le pourcentage en poids de tous les constituants du mélange dépolymérisé dans l'héparine de bas poids moléculaire de départ. Pour les 4 dérivés 1,6 anhydro qui correspondent aux pics 7,8,13 et 19, on obtient les pourcentages en poids suivants:

$$\% \, w/w_{7+8} = 100 \cdot \frac{443 \cdot (Area_7 + Area_8)}{\sum Mw_x \cdot Area_x} \quad ;$$

$$\% \, w/w_{13} = 100 \cdot \frac{545 \cdot Area_{13}}{\sum Mw_x \cdot Area_x}$$

$$\% \, w/w_{19} = 100 \cdot \frac{1210 \cdot Area_{13}}{\sum Mw_x \cdot Area_x}$$

$Area_7$, $Area_8$, $Area_{13}$ et $Area_{19}$ correspondent aux aires de chacun des pics 7,8,13 et 19. Les masses molaires de chacun de ces 4 composés sont respectivement 443, 443, 545 et 1210. $\Sigma Mw_x \cdot Area_x$ correspond au rapport de l'aire de chaque pic du chromatogramme par la masse molaire du produit correspondant.

Si $M_w$ est la masse moyenne de l'héparine de bas poids moléculaire étudiée, le pourcentage de chaînes oligosaccharidiques se terminant par un cycle 1,6 anhydro est obtenu de la façon suivante :

Les masses moléculaires des constituants sont les suivantes :

| Oligosaccharide | Oligosaccharide après réduction | Masse moléculaire |
|---|---|---|
| 1 | 1 | 741 |
| 2 | 20 | 401 |
| 3 | 3 | 734 |
| 4 | 21 | 461 |
| 5 | 22 | 461 |
| 6 | 23 | 503 |
| 7 | 7 | 443 |
| 8 | 8 | 443 |
| 9 | 24 | 503 |
| 10 | 25 | 563 |
| 11 | 26 | 563 |
| 12 | 27 | 563 |
| 13 | 13 | 545 |
| 14 | 28 | 605 |
| 15 | 29 | 1066 |
| 16 | 30 | 665 |
| 17 | 31 | 965 |
| 18 | 32 | 1168 |
| 19 | 19 | 1210 |

<u>**Nomenclature des saccharides et correspondance avec les pics selon les figures 1 et 2**</u>

IdoA : acide -α-L-Idopyranosyluronique;
GlcA: : acide -β-D-Glucopyranosyluronique;
ΔGlcA :acide 4,5-insaturaté : acide 4-déoxy-α-L-threo-hex-4-enepyranosyluronique
Gal : D-Galactose ;
Xyl : xylose ;
GlcNAc : 2-deoxy-2-acetamido-α-D-glucopyranose;
GlcNS : 2-deoxy-2-sulfamido-α-D-glucopyranose;

2S : 2-O sulfate,
3S : 3-O sulfate,
6S : 6-O sulfate

1 : ΔGlcA β1-3 Gal $\beta_{1-3}$ Gal $\beta_{1-4}$ Xyl$\beta_1$-O-Ser
2 : acide 4-désoxy-α-L-thréo-hex-enepyranosyluronique-(1→ 4)-2-désoxy-2-acétamido-α-D-glucopyranosyl sel de sodium
3 : ΔGlcA $\beta_{1-3}$ Gal $\beta_{1-3}$ Gal $\beta_{1-4}$ Xyl $\beta_1$-O-$CH_2$-COOH
4: acide 4-deoxy-α-L-threo-hex-4-enegalactopyranosyluronique-(1→ 4)-2-deoxy-2-sulfamido-β-D-glucopyranose sel de disodium
5: acide 4-désoxy-α-L-thréo-hex-enepyranosyluronique-(1→ 4)-2-désoxy-2-sulfamido- -α-D-glucopyranosyl sel de disodium
6: acide 4-désoxy-α-L-thréo-hex-enepyranosyluronique-(1→ 4)-2-désoxy-2-acétamido-6-O-sulfo-α-D-glucopyranosyl sel de disodium
7: acide 4-deoxy-α-L-threo-hex-4-enepyranosyluronique-(1→ 4)-1,6-anhydro-2-deoxy-2-sulfamido-β-D-glucopyranose sel de disodium (disaccharide 1)
8: acide 4-deoxy-α-L-threo-hex-4-enepyranosyluronique-(1→ 4)-1,6-anhydro-2-deoxy-2-sulfamido-β-D-mannopyranose sel de disodium (Disacharide 2)
9: acide 4-désoxy-2-O-sulfo-α-L-thréo-hex-enepyranosyluronique-(1→ 4)-2-désoxy-2-acétamido-α-D-glucopyranosyl sel de disodium
10: acide 4-deoxy-α-L-threo-hex-4-enegalactopyranosyluronique-(1→ 4)-2-deoxy-2-sulfamido-6-O-sulfo-β-D-glucopyranose sel de triisodium
11 : acide 4-désoxy-α-L-thréo-hex-enepyranosyluronique-(1→ 4)-2-désoxy-2-sulfamido-6-O-sulfo-α-D-glucopyranosyl sel de trisodium
12 : acide 4-désoxy-2-O-sulfo-α-L-thréo-hex-enepyranosyluronique-(1→ 4)-2-désoxy-2-sulfamido-α-D-glucopyranosyl sel de trisodium
13 : acide 4-deoxy-2-O-sulfo-α-L-threo-hex-4-enepyranosyluronique- (1→ 4)-1,6-anhydro-2-deoxy-2-sulfamido-β-D-glucopyranose sel de trisodium (Disaccharide 3)
14 : acide 4-désoxy-2-O-sulfo-α-L-thréo-hex-enepyranosyluronique-(1→ 4)-2-désoxy-2-acétamido-6-O-sulfo-α-D-glucopyranosyl sel de trisodium
15 : acide 4-désoxy -α-L-thréo-hex-enepyranosyluronique-(1→ 4)-2-désoxy-2-acétamido-6-O-sulfo-α-D-glucopyranosyl-(1→ 4)-acide β-D-glucopyranosyluronique-(1→ 4)-2-désoxy-2-sulfamido-3-O-sulfo-α-D-glucopyranosyl) sel de pentasodium
16 : acide 4-désoxy-2-O-sulfo-α-L-thréo-hex-enepyranosyluronique-(1→ 4)-2-désoxy-2-sulfamido-6-O-sulfo-α-D-glucopyranosyl sel de tétrasodium
17 : acide 4-désoxy-α-L-thréo-hex-enepyranosyluronique-(1→ 4)-2-désoxy-2-acétamido-6-O-sulfo-α-D-glucopyranosyl-(1→ 4)-acide β-D-glucopyranosyluronique-(→ 4)-2-désoxy-2-sulfamido-3,6-di-O-sulfo-α-D-glucopyranosyl) sel d'hexasodium
18 : acide 4-désoxy-2-O-sulfo-α-L-thréo-hexenepyranosyluronique-(1→ 4)-2-désoxy-2-sulfamido-6-O-sulfo-D-glucopyranosyl-(1→ 4)-acide-2-O-sulfo α-L-idopyranosyluronique sel d'hexasodium
19: acide 4-désoxy-2-O-sulfo-α-L-thréo-hex-enepyranosyluronique-(1→ 4)-2-désoxy-2-sulfamido-6-O-sulfo-α-D-glucopyranosyl-(1→ 4)-acide-2-O-sulfo α-L-idopyranosyluronique- (1→ 4)-1,6-anhydro-2-deoxy-2-sulfamido-β-D-mannopyranose, sel de heptasodium (tetrasaccharide 1)
20 : acide 4-désoxy-α-L-thréo-hex-enepyranosyluronique-(1→ 4)-2-désoxy-2-acétamido-α-D-glucitol sel de sodium
21 : acide 4-deoxy-α-L-threo-hex-4-enegalactopyranosyluronique- (1→ 4)- 2-deoxy-2-sulfamido-β-D-glucitol sel de disodium
22 : acide 4-désoxy-α-L-thréo-hex-enepyranosyluronique-(1→ 4)-2-désoxy-2-sulfamido-α-D-glucitol sel de disodium
23 : acide 4-désoxy-α-L-thréo-hex-enepyranosyluronique-(1→ 4)-2-désoxy-2-acétamido-6-O-sulfo-α-D-glucitol sel de disodium
24 : acide 4-désoxy-2-O-sulfo-α-L-thréo-hex-enepyranosyluronique- (1→ 4)-2-désoxy-2-acétamido-α-D-glucitol sel de disodium
25 : acide 4-deoxy-α-L-threo-hex-4-enegalactopyranosyluronique- (1→ 4)- 2-deoxy-2-sulfamido-6-O-sulfo-β-D-glucitol sel de triisodium
26 : acide 4-désoxy-α-L-thréo-hex-enepyranosyluronique-(1→ 4)-2-désoxy-2-sulfamido-6-O-sulfo-α-D-glucitol sel de trisodium
27 : acide 4-désoxy-2-O-sulfo-α-L-thréo-hex-enepyranosyluronique-(1→ 4)-2-désoxy-2-sulfamido-α-D-glucitol sel de trisodium

28 : acide 4-désoxy-2-O-sulfo-α-L-thréo-hex-enepyranosyluronique- (1→ 4)-2-désoxy-2-acétamido-6-O-sulfo-α-D-glucitol sel de trisodium

29 : acide 4-désoxy -α-L-thréo-hex-enepyranosyluronique-(1→ 4)-2-désoxy-2-acétamido-6-O-sulfo-α-D-glucopyranosyl-(1→ 4)-acide β-D-glucopyranosyluronique-(1→ 4)-2-désoxy-2-sulfamido-3-O-sulfo-α-D-glucitol) sel de pentasodium

30 : acide 4-désoxy-2-O-sulfo-α-L-thréo-hex-enepyranosyluronique- (1→ 4)-2-désoxy-2-sulfamido-6-O-sulfo-α-D-glucitol sel de tétrasodium

31 : acide 4-désoxy -α-L-thréo-hex-enepyranosyluronique-(1→ 4)-2-désoxy-2-acétamido-6-O-sulfo-α-D-glucopyranosyl-(1→ 4)-acide β-D-glucopyranosyluronique-(1→ 4)-2-désoxy-2-sulfamido-3,6-di-O-sulfo-α-D-glucitol) sel d'hexasodium

32 : acide 4-désoxy-2-O-sulfo-α-L-thréo-hexenepyranosyluronique-(1→ 4)-2-désoxy-2-sulfamido-6-0-sulfo-α-D-glucopyranosyl-(1→ 4)-acide -2-O-sulfo α-L-idopyranosyluronique sel d'hexasodium (forme réduite par $NaBH_4$).

Figure 1

Séparation chromatographique de l'Enoxaparine dépolymérisée par les héparinases avant et après réduction par $NaBH_4$ (Signal en noir fin : UV à 234nm ; Signal en noir épais UV : 202 - 234nm)

Figure 2 : Séparation chromatographique de l'héparine dépolymérisée par les héparinases avant et après réduction par $NaBH_4$ (Signal en noir fin : UV à 234nm ; Signal en noir épais : UV : 202 - 234nm)

## Revendications

**1.** Méthode d'analyse des héparines ou des héparines de bas poids moléculaire pour rechercher la présence de chaînes oligosaccharides dont la terminaison est modifiée par une liaison 1,6-anhydro, **caractérisée en ce qu'**on effectue les étapes suivantes :

1) dépolymérisation de l'échantillon par action d'héparinases,
2) réduction du dépolymérisat,
3) dosage par Chromatographie liquide Haute Performance, la méthode chromatographique utilisée étant une chromatographie par échange d'anion pour la détermination des groupements 1,6-anhydro, dans laquelle :

- on utilise une phase mobile transparente dans l'UV jusqu'à 200 nm et
- une double détection à 234 nm d'une part et 202-230 nm d'autre part permettant de détecter sélectivement les sucres acétylés, la dite méthode s'effectuant en prenant comme signal, pour la détection sélective des sucres acétylés, la différence entre l'absorbance à ces 2 longueurs d'onde (202 et 230 nm) choisies de telle manière que l'absorptivité des saccharides non acétylés s'annule,

et **caractérisée en ce que** les résidus 1,6-anhydro obtenus lors de la réaction de dépolymérisation sont les suivants :

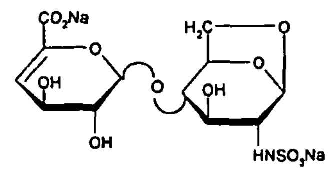

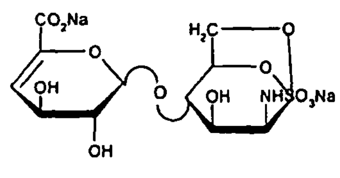

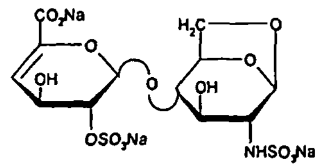

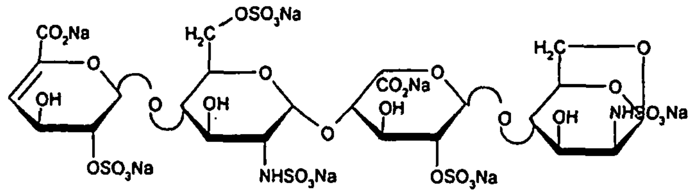

2. Méthode telle que définie à la revendication 1 **caractérisée en ce que** les héparinases sont sous forme d'un mélange d'héparinase 1 (EC 4.2.2.7.), d'héparinase 2 (heparin lyase II) et d'héparinase 3 (EC.4.2.2.8.)

3. Méthode telle que définie à la revendication 1 ou 2, **caractérisée en ce que** l'héparine dépolymérisée par action d'héparinase (dépolymérisat) est ensuite soumise à un agent de réduction.

4. Méthode telle que définie à la revendication 3, **caractérisée en ce que** l'agent de réduction est le $NaBH_4$ ou un sel de métal alcalin de l'anion borohydrure.

5. Méthode telle que définie dans l'une quelconque des revendications 1 à 4, **caractérisée en ce que** la phase mobile utilisée est à base de perchlorate de sodium, de sels de méthane sulfonate ou de sels de phosphate.

6. Méthode telle que définie dans l'une quelconque des revendications 1 à 5, **caractérisée en ce que** les 2 longueurs d'onde pour la détection des sucres acétylés sont de 202 et 230 nm.

7. Dérivé 1, 6 anhydro de formule (disaccharide 1) :

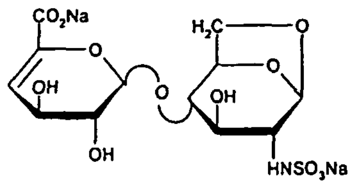

8. Dérivé 1,6 anhydro de formule (disaccharide 2) :

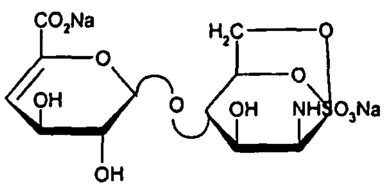

9. Dérivé 1,6 anhydro de formule (disaccharide 3) :

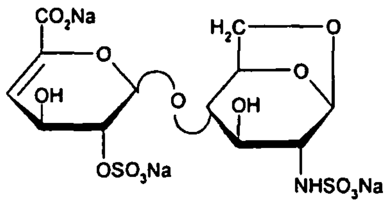

**10.** Dérivé trisaccharide de formule :

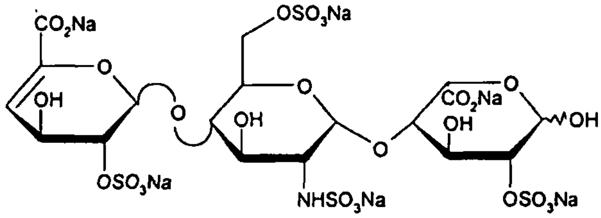

## Claims

**1.** Method for analyzing heparins or low-molecular-weight heparins for detecting the presence of oligosaccharide chains whose end is modified with a 1,6-anhydro bond, **characterized in that** the following steps are carried out:

1)- depolymerization of the sample by the action of heparinases
2)- reduction of the depolymerisate
3)- assay by High-Performance Liquid Chromatography, the chromatographic method used being an anion-exchange chromatography for the determination of 1,6-anhydro groups, in which

- a mobile phase that is transparent in UV is used up to 200 nm, and
- a double detection at 234 nm, on the one hand, and 202-230 nm, on the other hand, which makes it possible to selectively detect acetylated sugars is used, the said method being carried out taking, as signal, for the selective detection of acetylated sugars, the difference between the absorbance at these 2 wavelengths (202 and 230 nm) chosen such that the absorptivity of the non-acetylated saccharides is reduced to zero,

and **characterized in that** the 1,6-anhydro residues obtained during the depolymerization reaction are the following:

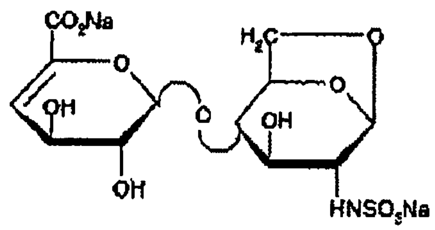

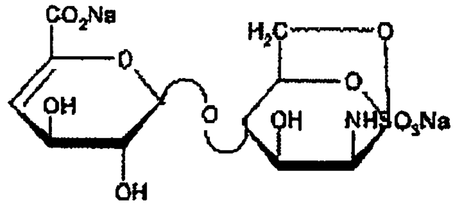

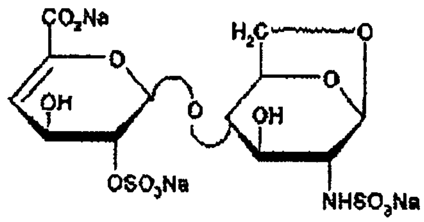

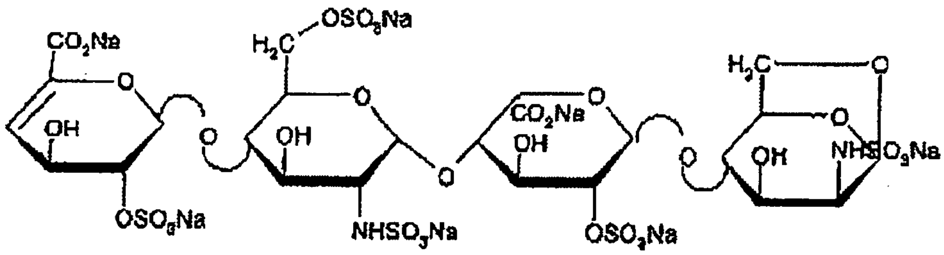

2. Method as defined in Claim 1, **characterized in that** the heparinases are in the form of a mixture of heparinase 1 (EC 4.2.2.7.), heparinase 2 (heparin lyase II) and heparinase 3 (EC.4.2.2.8.).

3. Method as defined in Claim 1 or 2, **characterized in that** the heparin depolymerized by the action of heparinase (depolymerisate) is then subjected to a reducing agent.

4. Method as defined in Claim 3, **characterized in that** the reducing agent is $NaBH_4$ or an alkali metal salt of the borohydride anion.

5. Method as defined in any one of Claims 1 to 4, **characterized in that** the mobile phase used is based on sodium perchlorate, methanesulphonate salts or phosphate salts.

6. Method as defined in any one of Claims 1 to 5, **characterized in that** the 2 wavelengths for the detection of the acetylated sugars are 202 and 230 nm.

7. 1,6-Anhydro derivative of formula (disaccharide 1):

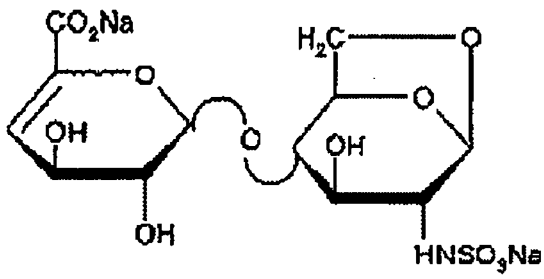

8. 1,6-Anhydro derivative of formula (disaccharide 2):

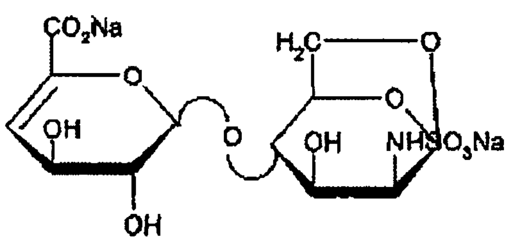

**9.** 1,6-Anhydro derivative of formula (disaccharide 3):

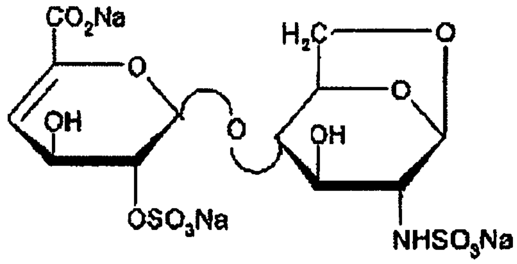

**10.** Trisaccharide derivative of formula:

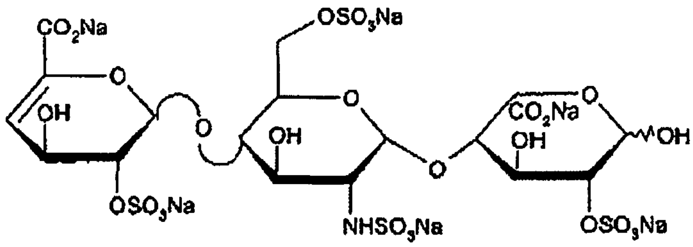

**Patentansprüche**

**1.** Verfahren für die Analyse von Heparinen oder Heparinen mit niedrigem Molekulargewicht für die Suche nach dem Vorhandensein von Oligosaccharidketten, deren Endgruppe durch eine 1,6-Anhydrobindung modifiziert ist, **dadurch gekennzeichnet, dass** man die folgenden Schritte durchführt:

1) Entpolymerisierung der Probe durch Einwirkung von Heparinasen,
2) Reduktion des Entpolymerisats,
3) quantitative Bestimmung mittels Hochdruckflüssigkeitschromatographie, wobei es sich bei der eingesetzten Chromatographiemethode um eine Anionenaustauschchromatographie für die Bestimmung der 1,6-Anhydrogruppen handelt, in der:

- man eine im UV bis 200 nm transparente mobile Phase und
- eine doppelte Detektion bei 234 nm sowie bei 202-230 nm, mit der man selektiv die acetylierten Zucker nachweisen kann, einsetzt, wobei das Verfahren dadurch durchgeführt wird, dass man als Signal für den selektiven Nachweis der acetylierten Zucker den Unterschied zwischen der Extinktion bei diesen zwei gewählten Wellenlängen (202 und 230 nm) wählt, so dass das Absorptionsvermögen der nichtacetylierten Saccharide verschwindet,

und **dadurch gekennzeichnet, dass** es sich bei den bei der Entpolymierisierung erhaltenen 1,6-Anhydroresten um die Folgenden handelt:

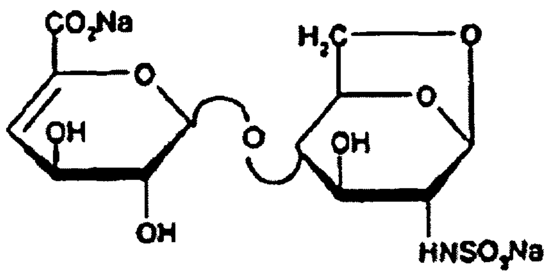

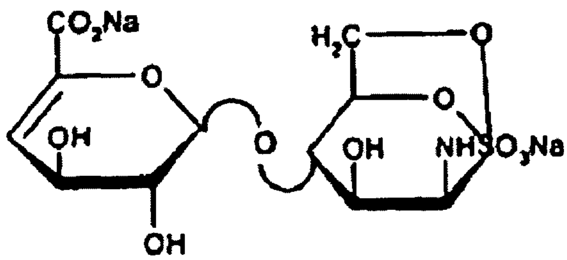

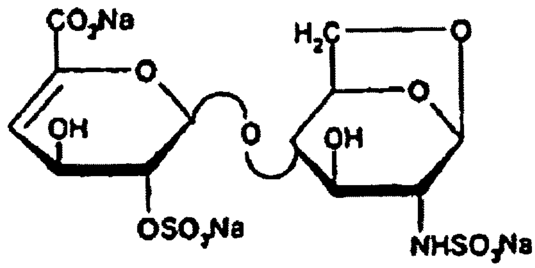

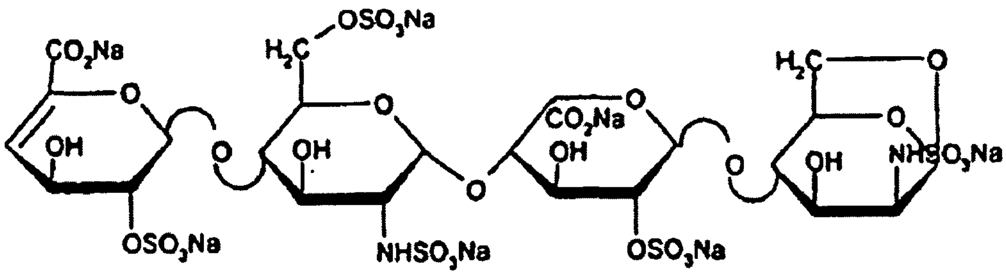

2. Verfahren nach Anspruch 1, **dadurch gekennzeichnet, dass** die Heparinasen in Form einer Mischung aus Heparinase 1 (EC 4.2.2.7.), Heparinase 2 (Heparinlyase II) und Heparinase 3 (EC.4.2.2.8) vorliegen.

3. Verfahren nach Anspruch 1 oder 2, **dadurch gekennzeichnet, dass** das durch Heparinasewirkung entpolymerisierte Heparin (Entpolymerisat) anschließend einem Reaktionsmittel ausgesetzt wird.

4. Verfahren nach Anspruch 3, **dadurch gekennzeichnet, dass** es sich bei dem Reduktionsmittel um $NaBH_4$ oder um ein Alkalimetallsalz des Borhydridanions handelt.

5. Verfahren nach einem der Ansprüche 1 bis 4, **dadurch gekennzeichnet, dass** die verwendete mobile Phase auf Natriumperchlorat, Methansulfonatsalzen oder Phosphatsalzen beruht.

6. Verfahren nach einem der Ansprüche 1 bis 5, **dadurch gekennzeichnet, dass** die zwei Wellenlängen für den Nachweis der acetylierten Zucker 202 und 230 nm betragen.

7. 1,6-Anyhdroderivat der Formel (Disaccharid 1):

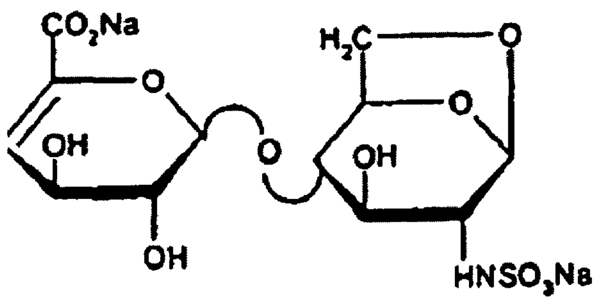

**8.** 1,6-Anyhdroderivat der Formel (Disaccharid 2):

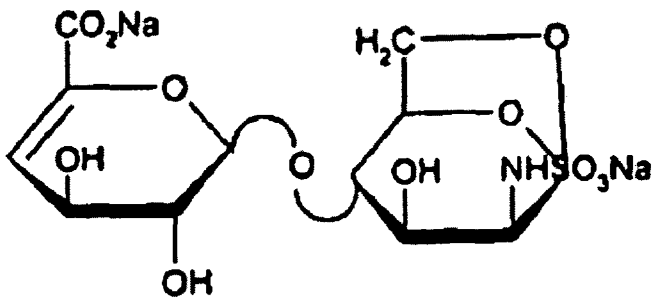

**9.** 1,6-Anyhdroderivat der Formel (Disaccharid 3):

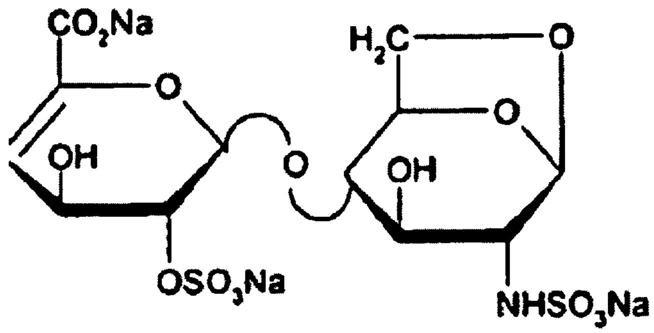

**10.** Trisaccharidderivat der Formel:

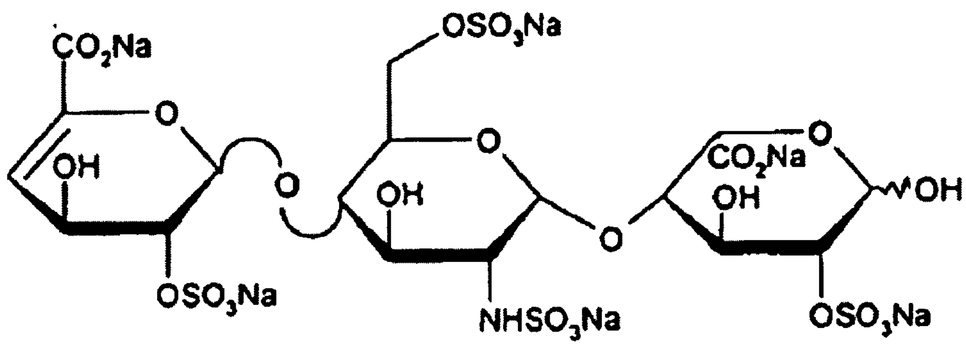

Figure 1

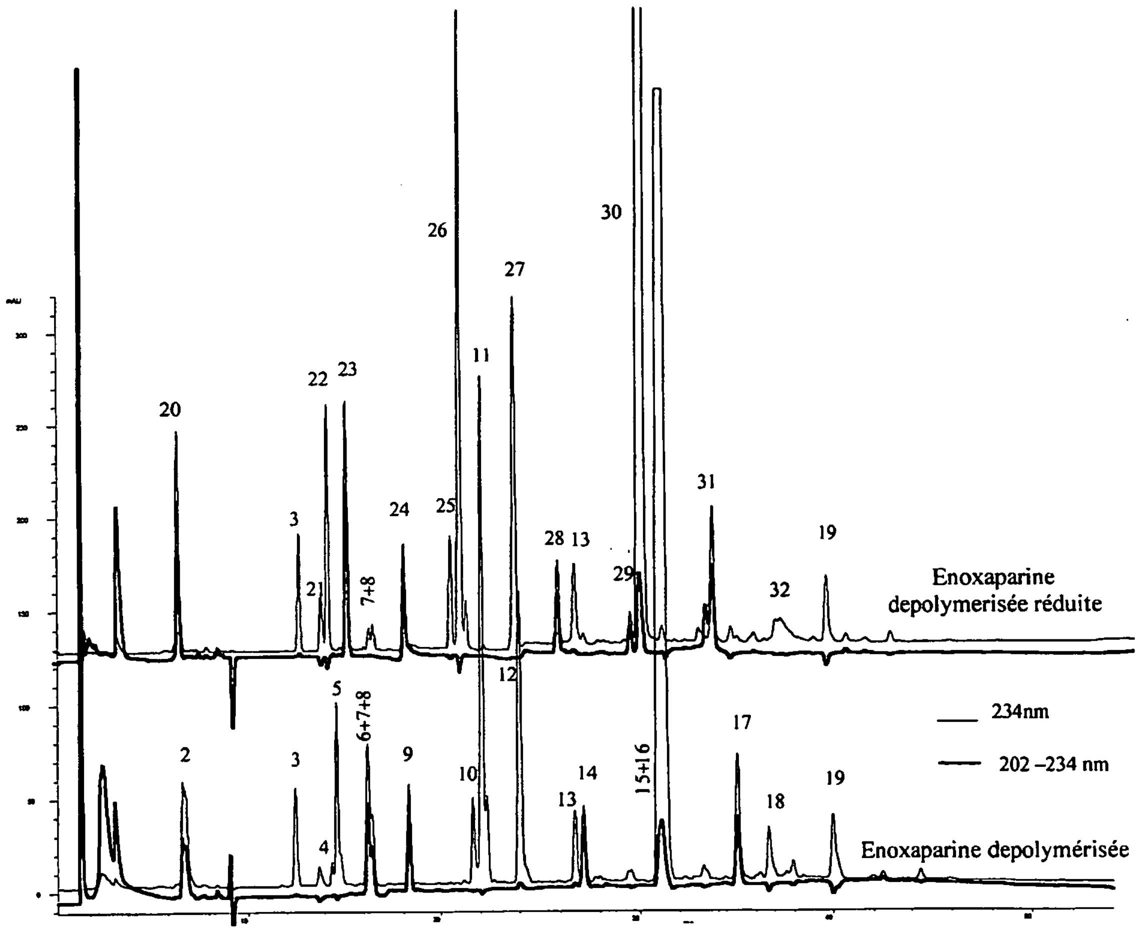

20
22
23
26
27
11
30
31
3
24
25
28 13
19
21
7+8
29
32
Enoxaparine depolymerisée réduite
12
5
6+7+8
234nm
202 -234 nm
2
3
9
10
14
15+16
17
18
19
13
4
Enoxaparine depolymérisée

Figure 2

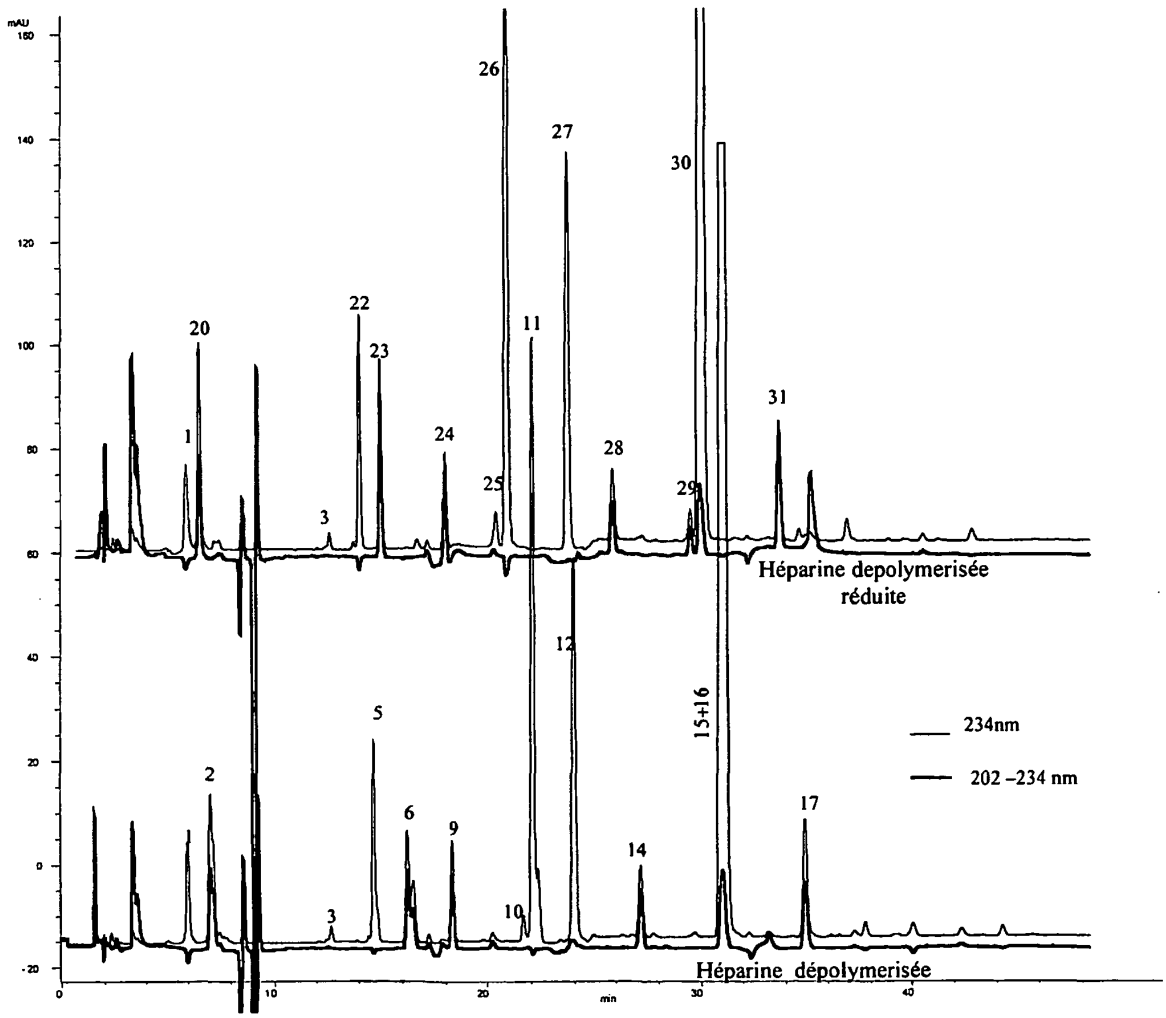
mAU
160
140
120
100
80
60
40
20
0
-20
0
10
20
min
30
40
26
27
30
22
20
23
11
31
1
24
28
25
29
3
Héparine depolymerisée réduite
12
15+16
5
234nm
202 -234 nm
2
17
6
9
14
3
10
Héparine dépolymerisée

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- US 5389618 A
- WO 0129055 A
- WO 8802400 A
- WO 0172762 A


### Littérature non-brevet citée dans la description


- **T. TOIDA.** *J. Carbohydrate Chemistry,* 1996, vol. 15 (3), 351-360
- **U.M. DESAI.** *Coll. Arch. Biochem. Biophys.,* 1993, vol. 306 (2), 461-468
- **S.YAMADA ; K.YOSHIDA ; M. SUGIURA ; K.SUGAHARA ; K-H KHOO ; H.R. MORRIS ; A. DELL.** *J.Biol.Chem.,* 1993, vol. 270 (7), 4780-4787
- **K.SUGAHARA ; H.TSUDA ; K.YOSHIDA ; S.YAMADA.** *J.Biol.Chem.,* 1995, vol. 270 (39), 22914-22923
- **K.SUGAHARA ; S.YAMADA ; K.YOSHIDA ; P. DE WAARD ; J.F.G. VLIEGENTHART.** *J.Biol.Chem.,* 1992, vol. 267 (3), 1528-1533